**Europäisches Patentamt**

**European Patent Office**

**Office européen des brevets**

(19)

(11) Veröffentlichungsnummer: **0 203 909**
**B1**

# (12) EUROPÄISCHE PATENTSCHRIFT

(45) Veröffentlichungstag der Patentschrift:
**02.11.89**

(21) Anmeldenummer: **86890109.1**

(22) Anmeldetag: **21.04.86**

(51) Int. Cl.⁴: **A61K 39/12, C12N 7/06**

(54) Frühsommer-Meningoenzephalitis-Virus(FSME-Virus)-Vakzine und Verfahren zu ihrer Herstellung.

(30) Priorität: **26.04.85 AT 1258/85**

(43) Veröffentlichungstag der Anmeldung:
**03.12.86 Patentblatt 86/49**

(45) Bekanntmachung des Hinweises auf die Patenterteilung:
**02.11.89 Patentblatt 89/44**

(84) Benannte Vertragsstaaten:
**CH DE FR GB IT LI NL**

(56) Entgegenhaltungen:
EP-A- 0 106 837
FR-A- 2 444 466
GB-A- 806 950

BIOLOGICAL ABSTRACTS, Band 80, Nr. 7, 1985,
Nr. 64096, M.P. CHUMAKOV et al.: "Subunit immunogen
of tick-borne encephalitis virus: Immunological
characteristics of V3 glycoprotein from 2 antigenic
types of tick-borne encephalitis virus" &VOPR
VIRUSOL 29(6): 701-706. 1984 000
JOURNAL OF CHROMATOGRAPHY, Band 297, 1984,
Seiten 63-73, Elsevier Science Publishers B.V.,
Amsterdam, NL; G. WINKLER et al.: "Exclusive use of
high-performance liquid chromatographic techniques
for the
isolation, 4-dimethylaminoazobenzene-4'-sulphonyl

(73) Patentinhaber: **IMMUNO Aktiengesellschaft für
chemisch-medizinische Produkte, Industriestrasse 72,
A-1220 Wien(AT)**

(72) Erfinder: **Heinz, Franz Xaver, Dr., Brühlerstrasse 114/9,
A-2340 Mödling(AT)**
Erfinder: **Kunz, Christian, Prof. Dr., Naaffgasse 71,
A-1180 Wien(AT)**

(74) Vertreter: **Wolfram, Gustav, Dipl.-Ing.,
Schwindgasse 7 P.O. Box 205, A-1041 Wien(AT)**

(56) Entgegenhaltungen: (Fortsetzung)
chloride amino acid analysis
and 4-N,N-dimethylaminoazobenzene-4'-Isothiocyana-
te-phenylisothiocyanate sequencing of a viral
membrane protein"
INFECTION AND IMMUNITY, Band 33, Nr. 1, Juli 1981,
Seiten 250-257; F.X. HEINZ et al.: "Antigenic and
immunogenic properties of defined physical forms of
tick-borne encephalitis virus structural proteins"

## Beschreibung

Die Erfindung betrifft eine Frühsommer-Meningoenzephalitis-Virus(FSME-Virus)-Vakzine.

Frühsommer-Meningoenzephalitis-Virus(FSME-Virus) ist ein pathogenes Flavi-Virus, welches durch den Biß infizierter Zecken auf den Menschen ubertragen wird. Charakteristische Krankheitssymptome sind Meningitis, Enzephalitis und Enzephalomyelitis; es kann auch zu Lähmungserscheinungen und Dauerschäden kommen. Die Letalitätsrate beträgt 1 bis 2 %.

Das FSME-Virus besitzt drei Strukturpolypeptide, die mit E, C und M bezeichnet werden (EP-A2 - 0 106 837), wobei das Polypeptid C im Nucleocapsid und die Polypeptide E und M gemeinsam mit Lipiden in der Virushülle enthalten sind. Das Genom des Virus besteht aus einer einzelstrangigen Ribonucleinsäure (RNA) mit Messenger-RNA-Polarität, welche infektiös ist und allein - ohne Zuhilfenahme viruseigener Enzyme - einen Infektionszyklus initiieren kann.

Nach der AT-B - 358.167 ist es bekannt, einen wirksamen Impfstoff gegen die Frühsommer-Meningoenzephalitis herzustellen, wobei eine auf Hühnerembryonalzellen gezüchtete Virussuspension durch eine Dichtegradienten-Ultrazentrifugation konzentriert, gereinigt und inaktiviert wird.

Es wurde gefunden, daß für die Induktion einer protektiven Immunität nicht alle im Virus vorhandenen Strukturproteine nötig sind. Darüber hinaus können Bestandteile lipidhältiger Viren Fieberreaktionen hervorrufen.

Die Erfindung bezweckt die Vermeidung solcher Nachteile und Schwierigkeiten und stellt sich die Aufgabe, FSME-Vakzine zur Verfügung zu stellen, die nicht aus inakti viertem Ganzvirus bestehen, sondern protektive subvirale Partikel in antigenreaktiver Form enthalten.

Diese Aufgabe wird erfindungsgemäß dadurch gelöst, daß die Frühsommer-Meningoenzephalitis-Virus(FSME-Virus)-Vakzine inaktiviertes und zumindest teilweise dissoziiertes FSME-Virus mit einem Gehalt an Glykoprotein E in immunogener Form enthält.

Vorzugsweise soll das Glykoprotein E in isolierter Form, d.h. physikalisch abgetrennt von den übrigen Proteinbestandteilen des Virus und vom Dissoziierungsmittel vorhanden sein.

Es ist zwar von F.X. Heinz, W. Tuma und Ch. Kunz in Infection and Immunity 33, 250-257 bereits über die Isolierung von Komponenten aus desintegriertem FSME-Virus berichtet worden, wobei die antigenen und immunogenen Eigenschaften der erhaltenen Glykoprotein-Komplexe mit denen des Gesamtvirus verglichen und gewisse Übereinstimmungen aufgezeigt wurden; jedoch wurden diese Komplexe nicht inaktiviert und für die Herstellung von Vakzinen in Betracht gezogen; die Aufspaltung bzw. Dissoziierung der Virusproteine ist für ihre Eignung bzw. Unbedenklichkeit in einer Vakzin-Präparation noch nicht ausreichend, zumal die Nucleinsäure allein, wie schon erwähnt, nach dem Eindringen in die Zelle einen Infektionszyklus initiieren kann.

Die erfindungsgemäße Frühsommer-Meningoenzephalitis-Virus-(FSME-Virus)-Vakzine ist weiters dadurch gekennzeichnet, daß

a) ihre antigene Wirksamkeit durch die Reaktivität mit polyklonalen und monoklonalen MausFSME-Antikörpern ausgedrückt ist und daß

b) ihre immunogene Wirksamkeit durch die Fähigkeit, nach Immunisierung von Versuchstieren virusspezifische Anti körper zu induzieren (Immunoassay-Test, Haemagglutinationshemmungstest), sowie durch die Fähigkeit, protektive Immunität zu induzieren, bestimmt ist.

Das Glykoprotein E liegt in den erfindungsgemäßen Präparationen in antigenreaktiver und immunogener Form vor. Aufgrund seiner Wanderungsgeschwindigkeit in der Natriumdodecylsulfat-(SDS)-Polyacrylamidgelelektrophorese (PAGE) (Lämmli u. Favre, 1973, J.Mol.Biol. 80, 575-599) besitzt das Glykoprotein E ein geschätztes Molekulargewicht von 50.000 bis 60.000.

Die Erfindung umfaßt des weiteren ein Verfahren zur Herstellung einer Vakzine und ist dadurch gekennzeichnet, daß eine Frühsommer-Meningoenzephalitis-Virus(FSME-Virus) enthaltende Suspension mit einem Detergens und/oder mit einem organischen Lösungsmittel behandelt wird, wobei es zu einer Dissoziierung des intakten Virus kommt, jedoch die Antigenreaktivität und Immunogenität des Glykoproteins E erhalten bleibt. Anschließend wird die so erhaltene Präparation inaktiviert und zu Impfstoffen aufgearbeitet.

Nach einer abgewandelten Ausführungsform ist das Verfahren dadurch gekennzeichnet, daß eine Frühsommer-Meningoenzephalitis-Virus(FSME-Virus) enthaltende Suspension inaktiviert, sodann mit einem Detergens und/oder mit einem organischen Lösungsmittel behandelt wird, wobei es zu einer Dissoziierung des inaktivierten Virus kommt, jedoch die Antigenreaktivität und Immunogenität des Glykoproteins E erhalten bleibt. Anschließend wird die so erhaltene Präparation zu Impfstoffen aufgearbeitet.

Als Detergens können nicht-ionische Detergentien, wie Triton X-100, Nonidet P-40 oder Octylglucosid verwendet werden. Es ist auch möglich, als Detergens ionische Detergentien, wie Cetyltrimethylammoniumbromid oder Desoxycho lat zu verwenden.

Das Detergens kann vor oder nach der Abtrennung der nicht-protektiven Proteinbestandteile durch Dialyse entfernt werden.

Vorzugsweise kann als Lösungsmittel Äther oder Tri-n-butylphosphat verwendet werden.

Nach bevorzugten Ausführungsformen des erfindungsgemäßen Verfahrens werden die nach den vorstehend beschriebenen Arbeitsweisen erhaltenen Präparationen zur Isolierung des protektiven Glykoproteins E einer weiteren Behandlung unterworfen, beispielsweise

a) einer Dichtegradienten-Ultrazentrifugation oder
b) einer Lectin-Affinitätschromatographie oder
c) einer Immuno-Affinitätschromatographie. um nicht-protektive Proteinbestandteile abzutrennen.

Bei diesen bevorzugten Ausführungsformen zur Isolierung des Glykoproteins E hängt die Wahl des Inaktivierungsmittels, namlich, ob man z.B. Formalin oder β-Propiolacton verwendet, davon ab, in welcher Phase des Verfahrens die Inaktivierung vorgenommen wird. Es hat sich herausgestellt, daß Formalin eine teilweise Vernetzung der Virusproteine verursacht, so daß eine vollkommene Dissoziierung nach einer Formalinbehandlung nicht mehr möglich ist. Eine Inaktivierung mit Formalin kann daher bei diesen bevorzugten Ausführungsformen nur als letzter Schritt vor der Aufarbeitung zu Impfstoffen angewendet werden, wogegen β-Propiolacton als Inaktivierungsmittel keine solche Vernetzung bewirkt. β-Propiolacton kann daher auch vor dem Dissoziierungsschritt als Inaktivierungsmittel eingesetzt werden. Letzteres wird bevorzugt, weil aufgrund der hohen Pathogenität des FSME-Virus eine möglichst frühe Inaktivierung einen produktionstechnischen Vorteil bringt.

Das erfindungsgemäße Verfahren und die Bestimmung der antigenen Wirksamkeit der hergestellten Präparationen ist in der folgenden detaillierten Beschreibung näher erläutert.

Als Ausgangsmaterial wird vorzugsweise eine auf Hühnerembryonalzellen gezüchtete Virussuspension verwendet. Es ist jedoch auch möglich, andere glykoproteinhältige Ausgangsmaterialien, z.B. virusinfizierte Zellen, als Ausgangsmaterial einzusetzen.

Beispiel 1:

Herstellung von Präparationen mit inaktiviertem und dissoziiertem FSME-Virus

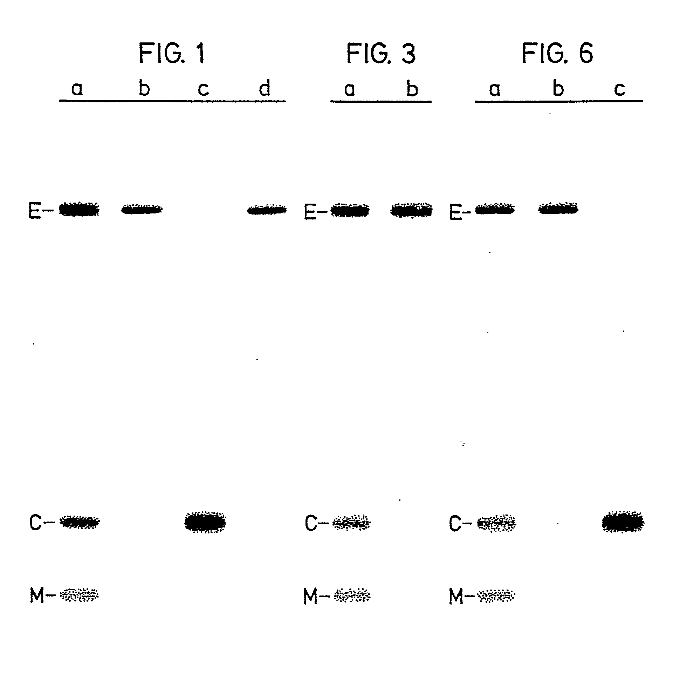

Eine durch Züchtung von FSME-Virus auf Hühnerembryonalzellen erhaltene Virussuspension wurde durch Ultrazentrifugieren konzentriert und durch zwei aufeinanderfolgende Zyklen einer Saccharosegradienten-Zentrifugation gereinigt, wie von Heinz und Kunz in J.gen.Virol. 57, 263-274, 1981, beschrieben. Eine mit dieser Suspension durchgeführte Natriumdodecylsulfat-Polyacrylamidgelelektrophorese unter Verwendung eines 15 %igen Gels (SDS-PAGE) zeigte das Vorhandensein der drei Virus-Strukturpolypeptide E, C und M, wie in Fig. 1a dargestellt.

Die gereinigte Virussuspension (40 µg/ml in Triäthanolaminpufferlösung, pH-Wert 8,0) wurde inaktiviert durch Behandlung mit

a) 0,05 % Formalin, 37°C, 24 Stunden bzw. mit
b) 0,025 % β-Propiolacton, 4°C, 22 Stunden und anschließend 37°C, 2 Stunden.

Die Dissoziierung dieser inaktivierten Viruspräparationen erfolgte durch Behandlung mit dem nichtionischen Detergens Triton X-100 bei 4°C, 1 Stunde (Verhältnis Detergens : Protein = 10 : 1). Die erhaltenen Suspensionen wurden zu Vakzinen weiterverarbeitet.

Beispiel 2:

Isolierung des Glykoproteins durch Dissoziation des Virus, Dichtegradientenzentrifugation und anschließende Inaktivierung

Eine durch Züchtung von FSME-Virus auf Hühnerembryonalzellen erhaltene Virussuspension wurde durch Ultrazentrifugieren konzentriert und durch zwei aufeinanderfolgende Zyklen einer Saccharosegradienten-Zentrifugation gereinigt, wie von Heinz und Kunz in J.gen.Virol. 57, 263-274, 1981, beschrieben. Eine mit dieser Suspension durchgeführte Natriumdodecylsulfat-Polyacrylamidgelelektrophorese unter Verwendung eines 15 %igen Gels (SDS-PAGE) zeigte das Vorhandensein der drei Virus-Strukturpolypeptide E, C und M, wie in Fig. 1a dargestellt.

Die gereinigte Virussuspension wurde mit einer Triäthanolaminpufferlösung auf eine Konzentration von 500 µg Virusprotein/ml und einen pH-Wert von 8,0 gestellt; Detergens Triton X-100 wurde bis zu einer Endkonzentration von 0,5 % zugegeben und die Mischung eine Stunde bei 4°C stehen gelassen, wobei eine Dissoziierung der Strukturpolypeptide erfolgte.

Die erhaltene Virus-Präparation wurde einer Dichtegradienten-Ultrazentrifugation unterworfen, indem sie auf einen linearen Saccharosegradienten (10 bis 40 % G/G) aufgetragen wurde, der am oberen Ende eine zusätzliche Schicht von 0,25 % Triton X-100 in 5 % Saccharose enthielt. Die Zentrifugation erfolgte bei 160.000 × g während 20 Stunden bei 4°C.

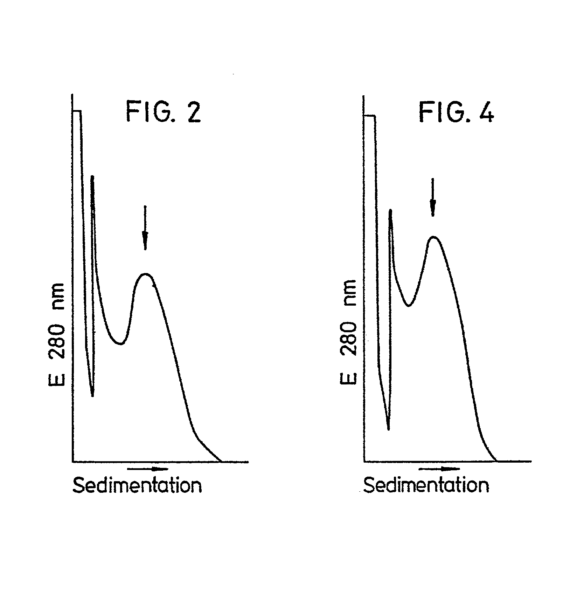

Nach dem Zentrifugieren wurde der Gradienteninhalt fraktioniert und die Extinktion bei 280 nm kontinuierlich aufgezeichnet, wie in Fig. 2 erläutert. Der mit dem Pfeil in Fig. 2 bezeichnete UV-Peak wurde gesammelt und einer weiteren SDS-PAGE-Analyse unterworfen, wobei das in Fig. 1b wiedergegebene Bandenbild erhalten wurde. In der gesammelten Fraktion war das Glykoprotein E enthalten, während das Nucleocapsid (Strukturpolypeptid C) unter den Zentrifugationsbedingungen pelletiert wurde. Die Analyse der Pellets mit Hilfe der SDS-PAGE ergab das Strukturpolypeptid C als einzigen Proteinbestandteil, wie aus Fig. 1c hervorgeht.

Obwohl das Strukturpolypeptid E an und für sich nicht infektiös ist, ist es nicht ratsam, aus der das Glykoprotein E enthaltenden Fraktion direkt eine Vakzine herzustellen, da die Dissoziierung allein keine ausreichende Verwendungssicherheit bietet. Dementsprechend wird erfindungsgemäß ein besonderer Inaktivierungsschritt vorgenommen, u.zw. entweder durch Behandlung mit Formalin oder durch Behandlung mit β-Propiolacton. Verwendet man Formalin, so ist eine Verdünnung von 1 : 2000, eine Temperatur von 37°C und eine Behandlungsdauer von 24 Stunden empfehlenswert; bei der Behandlung mit β-Propiolacton eine Verdünnung von 1 : 4000, eine Behandlungsdauer von 22 Stunden bei 4°C und anschließend 2 Stunden bei 37°C. Nach der Inaktivierung ergab sich bei der SDS-PAGE-Analyse das in Fig. 1d dargestellte Bandenbild, welches mit 1b übereinstimmt. Die inaktivierte Präparation wurde sodann zu Vakzinen weiterverarbeitet.

### Beispiel 3:

Isolierung des Glykoproteins durch Inaktivieren des Virus, Dissoziation und Dichtegradientenzentrifugation

Eine FSME-Virus-Suspension wurde in gleicher Weise, wie in Beispiel 1 beschrieben, bereitet und gereinigt. Die gereinigte Virussuspension wurde, wie ebenfalls in Beispiel 1 beschrieben, mit β-Propiolacton inaktiviert und damit eine SDS-PAGE-Analyse vorgenommen, wobei sich die in Fig. 3a dargestellte Verteilung der Strukturpolypeptide E und C ergab. Die inaktivierte Virussuspension wurde sodann mit dem Detergens Triton X-100 (Endkonzentration 0,5 %) behandelt und stehen gelassen, wobei die Strukturpolypeptide dissoziierten. Die dissoziierte Suspension wurde sodann einer Dichtegradientenzentrifugation, wie in Beispiel 2 beschrieben, unterworfen und dabei die in Fig. 4 dargestellte UV-Kurve erhalten.

Nach dem Zentrifugieren wurde der Gradienteninhalt fraktioniert und der mit dem Pfeil bezeichnete UV-Peak gesammelt. Die SDS-PAGE-Analyse dieses mit dem Pfeil bezeichneten UV-Peaks ist in Fig. 3b wiedergegeben; sie enthielt das von inaktiviertem Virus isolierte Glykoprotein E und wurde zu Vakzinen weiterverarbeitet.

Die nach den Beispielen 1, 2 und 3 hergestellten Präparationen liegen in einer Form vor, die das Glykoprotein E antigenreaktiv enthalten.

Die Intaktheit der Antigenstruktur wird durch folgende Versuche bewiesen:

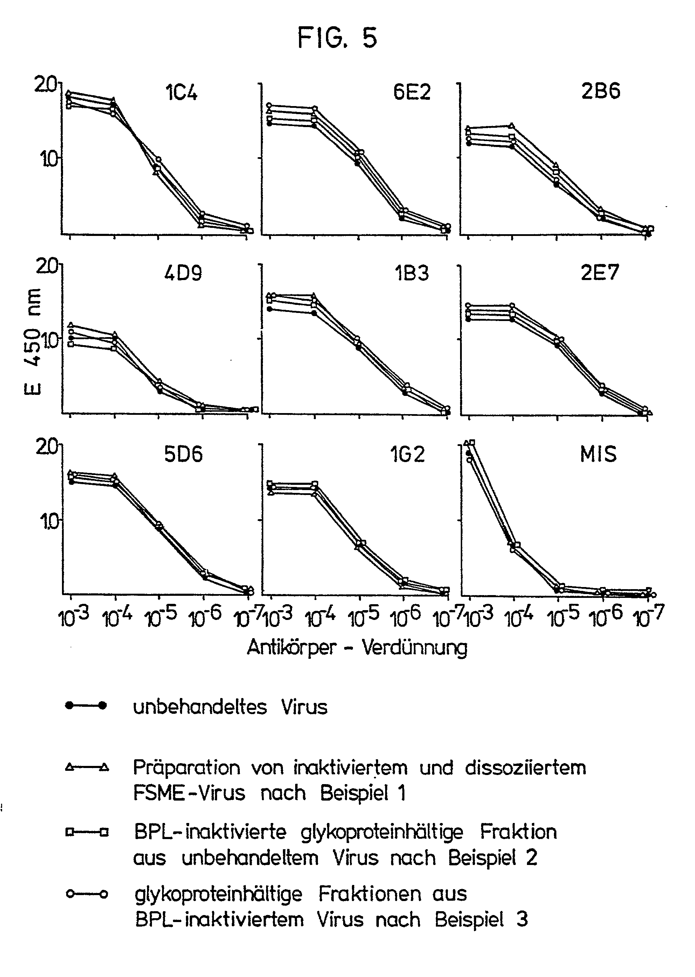

Proben der nach den Beispielen 1 und 2 hergestellten Glykoprotein-E-hältigen Präparationen wurden in einem Enzymimmunoassay auf ihre Reaktivität mit polyklonalen und monoklonalen Mausantikörpern, die nach Immunisierung mit dem nativen Glykoprotein erhalten worden waren, untersucht. Die zu vergleichenden Proben wurden in gleicher Proteinkonzentration (2 µg/ml) an Polystyrol-Mikrotiterplatten adsorbiert und dann, wie von Heinz et al. in Virology 126, 525-537, 1983, sowie in der EP-A2 - 0 106 837 beschrieben, mit Verdünnungsreihen des polyklonalen MIS und von acht genau charakterisierten monoklonalen Antikörpern 1C4, 6E2, 2B6, 4D9, 1B3, 2E7, 5D6, 1G2 zur Reaktion gebracht. Wie aus der Fig. 5 hervorgeht, ergeben erfindungsgemäß hergestellte Präparationen praktisch identische Titrationskurven wie nicht-inaktivierte glykoproteinhältige Präparationen oder das native Ganzvirus, welches das Glykoprotein als Bestandteil der Virusmembran enthält. Diese Ergebnisse beweisen, daß die erfindungsgemäßen Präparationen eine intakte Antigenstruktur besitzen.

### Beispiel 4:

Isolierung des Glykoproteins durch Dissoziation des Virus, Entfernung des Detergens durch Dialyse, Inaktivierung und Abtrennung des Nucleocapsids durch Ultrazentrifugation

Eine gereinigte Suspension von FSME-Virus mit einem Gehalt von 200 µg Protein/ml in Triäthanolaminpuffer bei einem pH-Wert von 8,0 wurde zur Solubilisierung der Lipidmembran mit dem dialysierbaren Detergens Octylglucosid bis zu einer Endkonzentration von 1 % versetzt und 1 Stunde bei Raumtemperatur stehen gelassen. Das Detergens wurde anschließend durch Dialyse gegen Triäthanmolaminpuffer bei einem pH-Wert von 8,0 entfernt und die Präparation wurde durch Zugabe von β-Propiolacton (1 : 4000) vollständig inaktiviert. Eine SDS-PAGE-Analyse wurde mit der Präparation durchgeführt, deren Ergebnis in Fig. 6a dargestellt ist. Wie ersichtlich sind alle drei Strukturproteine des Virus vorhanden.

Da für die Induktion einer protektiven Immunität das Nucleocapsid, bestehend aus der Virus Ribonucleinsäure und dem mit C bezeichneten Protein, nicht erforderlich ist, wurde dieses durch Ultrazentrifugieren wie folgt entfernt: In einem 5 ml Zentrifugenröhrchen wurde ein Zuckerpolster, bestehend aus 1 ml 40 %iger Saccharose in Triäthanolaminpuffer, pH-Wert 8,0, mit 1 ml der oben beschriebenen Probe überschichtet und 3 Stunden bei 120.000 × g/4°C zentrifugiert. Wie die in Fig. 6b dargestellte SDS-PAGE-Analyse zeigte, wurde dadurch das Nucleocapsid vollständig aus der glykoproteinhältigen Probe entfernt. Da das Virus-Core durch Octylglucosid nicht dissoziiert wird, wandert es durch den Saccharosepolster in das Pellet, in dem daher das Nucleocapsid C den einzigen Proteinanteil darstellt. Dies ist in Fig. 6c erläutert.

Der Nachweis der Intaktheit der Antigenstruktur der auf diese Weise hergestellten glykoproteinhältigen Präparation erfolgte wieder nach dem gleichen Verfahren, wie vorher beschrieben. Auch die Antigenreaktivität des Glykoproteins E in der in beschriebener Weise hergestellten Präparation war identisch mit jener des nativen Glykoproteins als Bestandteil des nichtinaktivierten Ganzvirus.

Beispiel 5:

Isolierung des Glykoproteins durch Lectin-Affinitätschromatographie und Inaktivierung

Da das für die Immunisierung essentielle Glykoprotein E das einzige glykosylierte Protein des FSME-Virus darstellt, kann dieses nach Dissoziation des Virus auch durch Lectin-Affinitätschromatographie von den für die Immunisierung nicht essentiellen Bestandteilen getrennt werden.

1 ml FSME-Virus (200 μg/ml) wurde durch Zugabe von Triton X-100 (0,2 % Endkonzentration) gespalten und mit 1 ml CONA Sepharose (Pharmacia, Uppsala) versetzt. Die nichtglykosylierten und daher nichtgebundenen Virusproteine wurden durch Waschen mit phosphatgepufferter Salzlösung, pH-Wert 7,4, entfernt, und die spezifische Elution des Glykoproteins E erfolgte mit Hilfe von 0,5 Mα-D Glukopyranosid. Nach Inaktivierung mit β-Propiolacton zeigte die Analyse mit Hilfe von Enzymimmunoassays, daß diese Präparation sowohl mit den polyklonalen als auch mit den monoklonalen Antikörpern reagierte und daher eine intakte Antigenstruktur besitzt.

Im folgenden Versuch wurden erfindungsgemäß hergestellte Präparationen zur Immunisierung verwendet:

Folgende Präparationen wurden zur Immunisierung von Mäusen verwendet:

a) Präparation von inaktiviertem und dissoziiertem FSME-Virus, wie in Beispiel 1a beschrieben.
b) Präparation von inaktiviertem und dissoziiertem FSME-Virus, wie in Beispiel 1b beschrieben.
c) Glykoprotein-E-hältige Präparation wie in Beispiel 2 beschrieben, inaktiviert mit Formalin.
d) Glykoprotein-E-hältige Präparation wie in Beispiel 2 beschrieben, jedoch inaktiviert mit β-Propiolacton.
e) Glykoprotein-E-hältige Präparation wie in Beispiel 3 beschrieben.
f) Glykoprotein-E-hältige Präparation wie in Beispiel 2 beschrieben, jedoch ohne Inaktivierung.

Mit jeder dieser Präparationen, die in einer Proteinkonzentration von 40 g/ml vorlagen, wurden 10 Mäuse mit einem Gewicht von 15 g zweimal im Abstand von 14 Tagen subcutan immunisiert. Die Dosis pro Immunisierung und Maus betrug 0,2 ml. 7 Tage nach der zweiten Immunisierung wurde den Mäusen Blut abgenommen, gepoolt und auf das Vorhandensein FSME-Virus-spezifischer Antikörper im Enzymimmunoassay und im Hämagglutinationshemmungstest analysiert (Heinz et al., Virology 126, 525-537, 1983). Am selben Tag wurden die Mäuse mit einer tödlichen Dosis (100 i.p. LD$_{50}$) FSME-Virus infiziert, um die protektive Wirkung der zur Impfung verwendeten Präparationen nachzuweisen.

Die Ergebnisse sind in der folgenden Tabelle zusammengefaßt. Daraus geht hervor, daß die erfindungsgemäßen Präparationen in den Mäusen Antikörper induzierten, die mit dem Glykoprotein als Bestandteil des ganzen Virus im Enzymimmunoassay reagierten, und auch die Hämagglutinations aktivität des Virus hemmten. Der Großteil der Mäuse war auch gegen eine ansonsten tödliche Infektion mit dem FSME-Virus geschützt.

**Tabelle**

| a Präparation | b Titer im HHt | c ELISA Einheiten | d e Mäuseschutzversuch |
|---|---|---|---|
| A | 160 | 180 | 10/10 |
| B | 40 | 65 | 8/10 |
| C | 60 | 75 | 9/10 |
| D | 160 | 170 | 9/10 |
| E | 160 | 180 | 8/10 |
| F | 120 | 180 | 9/10 |

a A: Präparation von inaktiviertem und dissoziiertem FSME-Virus laut Beispiel 1a
B: Präparation von inaktiviertem und dissoziiertem FSME-Virus laut Beispiel 1b
C: Glykoprotein-E-hältige Präparation laut Beispiel 2, inaktiviert mit Formalin
D: Glykoprotein-E-hältige Präparation laut Beispiel 2, inaktiviert mit β-Propiolacton
E: Glykoprotein-E-hältige Präparation laut Beispiel 3
F: Nicht-inaktivierte Kontrollpräparation laut Beispiel 2
b HHT = Hämagglutinationshemmtest

c ELISA-Einheiten: Ergebnis des Immunoassays ausgedrückt als willkürliche Einheit unter Verwendung eines Standardserums (Hofmann et al., Zbl. Bakt.Hyg., I.Abt.Orig. A255, 448-455, 1983)
  d Anzahl der überlebenden Mäuse
  e Anzahl der getesteten Mäuse

**Patentansprüche**

1. Frühsommer-Meningoenzephalitis-Virus(FSME-Virus)-Vakzine, dadurch gekennzeichnet, daß sie inaktiviertes und zumindest teilweise dissoziiertes FSME-Virus mit einem Gehalt an Glykoprotein E in immunogener Form enthält.
2. Vakzine nach Anspruch 1, dadurch gekennzeichnet, daß sie das Glycoprotein E in isolierter Form, d.h. abgetrennt von den übrigen Proteinbestandteilen des Virus und vom Dissoziierungsmittel enthält.
3. Vakzine nach Anspruch 1 oder 2, dadurch gekennzeichnet, daß
a) ihre antigene Wirksamkeit durch die Reaktivität mit polyklonalen und monoklonalen MausFSME-Antikörpern ausgedrückt ist und daß
b) ihre immunogene Wirksamkeit durch die Fähigkeit, nach Immunisierung von Versuchstieren virusspezifische Antikörper zu induzieren (Immunoassay-Test, Haemagglutinationshemmungstest), sowie durch die Fähigkeit, protektive Immunität zu induzieren, bestimmt ist.
4. Verfahren zur Herstellung einer Vakzine nach den Ansprüchen 1 bis 3, dadurch gekennzeichnet, daß eine Frühsommer-Meningoenzephalitis-Virus(FSME-Virus) enthaltende Suspension mit einem Detergens und/oder mit einem organischen Lösungsmittel behandelt, sodann inaktiviert und zu Impfstoffen aufgearbeitet wird.
5. Verfahren zur Herstellung einer Vakzine nach den Ansprüchen 1 bis 3, dadurch gekennzeichnet, daß eine Frühsommer-Meningoenzephalitis-Virus(FSME-Virus) enthaltende Suspension inaktiviert, sodann mit einem Detergens und/oder mit einem organischen Lösungsmittel behandelt und zu Impfstoffen aufgearbeitet wird.
6. Verfahren nach Anspruch 4 oder 5, dadurch gekennzeichnet, daß als Detergens nicht-ionische Detergentien, wie Triton® X-100, Nonidet® P-40 oder Octylglucosid verwendet werden.
7. Verfahren nach Anspruch 4 oder 5, dadurch gekennzeichnet, daß als Detergens ionische Detergentien, wie Cetyltrimethylammoniumbromid oder Desoxycholat verwendet werden.
8. Verfahren nach den Ansprüchen 4 bis 7, dadurch gekennzeichnet, daß dialysierbare Detergentien, wie Octylglucosid, vor oder nach der Abtrennung der nicht-protektiven Proteinbestandteile durch Dialyse entfernt werden.
9. Verfahren nach Anspruch 4 oder 5, dadurch gekennzeichnet, daß als Lösungsmittel Äther oder Tri-n-butylphosphat verwendet werden.
10.Verfahren nach Anspruch 4 oder 5, dadurch gekennzeichnet, daß das protektive Glykoprotein E aus der dissoziierten, Virus enthaltenden Präparation isoliert wird, wobei nicht-protektive Proteinbestandteile abgetrennt werden, vorzugsweise mit Hilfe
a) einer Dichtegradienten-Ultrazentrifugation oder
b) einer Lectin-Affinitätschromatographie oder
c) einer Immuno-Affinitätschromatographie.
11.Verfahren nach Anspruch 4, dadurch gekennzeichnet, ,daß Formalin oder β-Propiolacton als Inaktivierungsmittel verwendet wird.
12.Verfahren nach Anspruch 5 und 10, dadurch gekennzeichnet, daß β-Propiolacton als Inaktivierungsmittel verwendet wird.

**Claims**

1. Early-summer meningo-encephalitis virus (FSME virus) vaccine, characterised in that it contains an inactivated and at least partially dissociated FSME virus having a content of glycoprotein E in immunogenic form.
2. Vaccine according to claim 1, characterised in that it contains glycoprotein E in isolated form, i.e., separated from the remaining protein components of the virus and from the dissociating agent.
3. Vaccine according to claim 1 or 2, characterised in that
a) its antigenic activity is expressed by the reactivity with polyclonal and monoclonal FSME antibodies of mice and
b) its immunogenic activity is determined by the ability to induce virus-specific antibodies after immunization of test animals (immunoassay, haemagglutination inhibition assay) as well as by its ability to induce protective immunity.
4. A method of producing a vaccine according to claims 1 to 3, characterised in that a suspension containing early-summer meningo-encephalitis virus (FSME virus) is treated with a detergent and/or with an organic solvent, then is inactivated and processed to vaccines.
5. A method of producing a vaccine according to claims 1 to 3, characterised in that a suspension containing early-summer meningo-encephalitis virus (FSME virus) is inactivated, then is treated with a detergent and/or an organic solvent and is processed to vaccines.

6. A method according to claim 4 or 5, characterised in that non-ionic detergents, such as Triton® X–100, Nonidet® p–40 or octyl glucoside are used as detergents.

7. A method according to claim 4 or 5, characterised in that ionic detergents, such as cetyl trimethyl ammonium bromide or desoxycholate, are used as detergents.

8. A method according to claims 4 to 7, characterised in that dialysable detergents, such as octyl glucoside, are eliminated by dialysis prior to or after the separation of non-protective protein components.

9. A method according to claim 4 or 5, characterised in that ether or tri-n-butyl phosphate is used as the solvent.

10. A method according to claim 4 or 5, characterised in that the protective glycoprotein E is isolated from the dissociated virus-containing preparation, wherein non-protective protein components are separated, preferably by the aid of
a) density gradient ultracentrifugation or
b) lectin affinity chromatography or
c) immuno affinity chromatography.

11. A method according to claim 4, characterised in that formalin or β-propiolactone is used as the inactivating agent.

12. A method according to claim 5 and 10, characterised in that β-propiolactone is used as the inactivating agent.

## Revendications

1. Vaccin contre le virus de la méningo-encéphalite du début de l'été (virus FSME), caractérisé en ce qu'il contient un virus FSME inactivé et au moins partiellement dissocié contenant la glucoprotéine E sous forme immunogène.

2. Vaccin selon la revendication 1, caractérisé en ce qu'il contient la glucoprotéine E sous forme isolée, c'est-à-dire séparée des autres constituants protéiniques du virus et de l'agent de dissociation.

3. Vaccin selon la revendication 1 ou 2, caractérisé en ce que
a) son activité antigénique est exprimée par la réactivité avec les anticorps FMSE de souris polyclonaux et monoclonaux et en ce que
b) son activité immunogène est déterminée par l'aptitude à induire après immunisation des animaux d'essai des anticorps spécifiques du virus (test de dosage immunologique, test d'anti-hémagglutination), ainsi que par l'aptitude à induire l'immunité protectrice.

4. Procédé pour la fabrication d'un vaccin selon les revendications 1 à 3, caractérisé en ce que l'on traite une suspension contenant le virus de la méningo-encéphalite du début de l'été (virus FSME) par un détergent et/ou par un solvant organique, ensuite on l'inactive et on la transforme en inoculants.

5. Procédé pour la fabrication d'un vaccin selon les revendications 1 à 3, caractérisé en ce que l'on inactive une suspension contenant le virus de la méningo-encéphalite du début de l'été (virus FSME), ensuite on la traite par un détergent et/ou par un solvant organique et on la transforme en inoculants.

6. Procédé selon la revendication 4 ou 5, caractérisé en ce que l'on utilise comme détergents des détergents non ioniques tels que® Triton X–100, Nonidet® P–40 ou octylglycoside.

7. Procédé selon la revendication 4 ou 5, caractérisé en ce que l'on utilise comme détergents des détergents ioniques tels que bromure de cétyltriméthylammonium ou désoxycholate.

8. Procédé selon les revendications 4 à 7, caractérisé en ce que l'on sépare par dialyse les détergents dialysables tels que l'octylglucoside avant ou après la séparation des constituants protéiniques non protecteurs.

9. Procédé selon la revendication 4 ou 5, caractérisé en ce que l'on utilise comme solvant l'éther ou le phosphate de tri-n-butyle.

10. Procédé selon la revendication 4 ou 5, caractérisé en ce que l'on isole la glucoprotéine E protectrice de la préparation contenant le virus dissocié en séparant les constituants protéiniques non protecteurs, de préférence
a) par une ultracentrifugation avec gradient de densité ou
b) par une chromatographie d'affinité sur lectine ou
c) par une chromatographie d'immuno-affinité.

11. Procédé selon la revendication 4, caractérisé en ce que l'on utilise comme agent d'inactivation la solution de fomaldéhyde ou la β-propiolactone.

12. Procédé selon les revendications 5 et 10, caractérisé en ce que l'on utilise comme agent d'inactivation la β-propiolactone.

FIG. 1
a b c d

FIG. 3
a b

FIG. 6
a b c

FIG. 2

E 280 nm

Sedimentation

FIG. 4

E 280 nm

Sedimentation

## FIG. 5